# EUROPEAN PATENT APPLICATION

(11) **EP 4 837 516 A1**
(43) Date of publication of application: **11.11.2026**
(21) Application number: 26169458.2
(22) Date of filing: 31.03.2026
(51) Int. Cl.: A61B 17/10, A61B 17/00, A61B 17/122

(54) **ASSEMBLY DEVICE, HEMOSTATIC CLIP KIT AND ASSEMBLY METHOD**

(30) Priority: 09.05.2025 CN 202510600388
(71) Applicant: Micro-Tech (Nanjing) Co., Ltd., Nanjing, Jiangsu 210032 (CN)
(72) Inventor: TANG, Zhi, Nanjing, 210032 (CN); LI, Chao, Nanjing, 210032 (CN)
(74) Representative: Kohler Schmid Möbus Patentanwälte

(57) **Abstract**

An assembly device, a hemostatic clip kit and an assembly method are disclosed. The assembly device has a housing assembly internally defining a receiving slot and an assembly slot. The receiving slot is configured to accommodate the clip assembly, and the assembly slot is configured to allow the delivery device to be inserted into the receiving slot via the assembly slot. Its positioning assembly is connected to the housing assembly and includes a first positioning part and a second positioning part disposed opposite to each other. The first and the second positioning parts are disposed between the receiving slot and the assembly slot, and are configured to engage with the mating part when the delivery device is inserted into the receiving slot. Through the above solution, during the assembly of the delivery device and the clip assembly, the delivery device can be inserted into the receiving slot via the assembly slot. The first and second positioning parts cooperate with the mating part of the delivery device to position the delivery device, ensuring accurate alignment with the clip assembly, thereby improving the efficiency of assembling the delivery device and the clip assembly and facilitating use.

## Description

The present disclosure claims the priority of Chinese application No. 202510600388.7 filed on May 9, 2025, and the entire contents of which are incorporated herein by reference.

### FIELD OF THE INVENTION

The present disclosure belongs to the field of medical instrument, and in particular relates to an assembly device, a hemostatic clip kit, and an assembly method.

### BACKGROUND OF THE INVENTION

Endoscopic hemostatic clips are a common medical hemostasis device. These products typically consist of two parts: a clip assembly and a delivery device. The clip assembly is used to clamp the bleeding site to achieve hemostasis, while the delivery device is used to deliver the clip assembly to the designated location within the body. Because the clip assembly is relatively small and needs to be used only once, it is usually housed in a separate housing. The clip assembly is pre-assembled and secured, and then the delivery device is inserted into the housing to complete the product assembly.

However, the current assembly method makes it difficult to accurately align the delivery device with the clip assembly, which affects assembly efficiency.

### SUMMARY OF THE INVENTION

Embodiments of the present disclosure provide an assembly device, a hemostatic clip kit, and an assembly method, aiming to solve the technical problem that current assembly methods are difficult to accurately align the delivery device and the clip assembly.

To achieve the above objective, according to a first aspect of the present disclosure, an assembly device is provided for assembling the clip assembly and the delivery device provided with a mating part, and the assembly device includes:
a housing assembly, internally defining a receiving slot and an assembly slot, wherein the receiving slot is configured to accommodate the clip assembly, and the assembly slot is configured to allow the delivery device to be inserted into the receiving slot via the assembly slot; and
a positioning assembly connected to the housing assembly, comprising a first positioning part and a second positioning part arranged opposite to each other, wherein the first positioning part and the second positioning part are disposed between the receiving slot and the assembly slot, and are configured to engage with the mating part when the delivery device is inserted into the receiving slot.

In some embodiments, the positioning assembly further includes a first elastic part and a second elastic part, wherein the first positioning part is connected to the housing assembly through the first elastic part, and the second positioning part is connected to the housing assembly through the second elastic part.

When the positioning assembly is subjected to pressure, the first elastic part and the second elastic part undergo elastic deformation, so that the first positioning part and the second positioning part are able to move.

In some embodiments, the first positioning part and/or the second positioning part have a first chamfered bevel on the side near the assembly slot.

When the delivery device moves from the assembly slot to the receiving slot, the first chamfered bevel is able to contact the delivery device.

In some embodiments, the first positioning part and/or the second positioning part has a second chamfered bevel on the side near the receiving slot.

When the delivery device moves from the receiving slot toward the assembly slot, the second chamfered bevel is able to contact the delivery device.

In some embodiments, the housing assembly includes a first housing and a second housing connected to the first housing.

In some embodiments, the first housing includes a first bearing part and a second bearing part, the receiving slot is disposed in the first bearing part, the assembly slot is disposed in the second bearing part, and the first positioning part and the second positioning part are disposed between the first bearing part and the second bearing part.

In some embodiments, the arrangement direction of the first bearing part and the second bearing part is a first direction, and the first positioning part and the second positioning part are disposed opposite to each other in a second direction, wherein the first direction and the second direction are perpendicular to each other.

The first elastic part and the second elastic part are respectively disposed on both sides of the first bearing part in the second direction, or the first elastic part and the second elastic part are respectively disposed on both sides of the second bearing part in the second direction.

In the second direction, both sides of the first elastic part and the second elastic part have movement clearances.

In some embodiments, when the positioning assembly is subjected to pressure exerted by the delivery device, the first positioning part and the second positioning part are able to move away from each other in the second direction.

In some embodiments, the second housing and the positioning assembly are disposed opposite each other in a third direction, and the first direction, the second direction, and the third direction are perpendicular to each other.

The second housing includes a pressing part, which, when pressed, is able to move toward the positioning assembly and apply pressure to the positioning assembly, so that the first positioning part and the second positioning part move away from each other in the second direction.

In some embodiments, the pressing part is provided with a first boss and a second boss facing the positioning assembly.

When the pressing part moves toward the positioning assembly, the first boss and the second boss move between the first positioning part and the second positioning part, and the first boss press the first positioning part, and the second boss press the second positioning part.

In some embodiments, the first boss has a first contact surface for contacting and pressing the first positioning part, the first contact surface being an inclined surface or an arc surface.

The second boss has a second contact surface for contacting and pressing the second positioning part, the second contact surface being an inclined surface or an arc surface.

In some embodiments, at least a portion of the first positioning part protrudes toward the pressing part relative to the first elastic part, and at least a portion of the second positioning part protrudes toward the pressing part relative to the second elastic part.

When the pressing part moves toward the positioning assembly, the first boss is able to contact the protruding part of the first positioning part, and the second boss is able to contact the protruding part of the second positioning part.

In some embodiments, the first housing is connected to the first elastic part, the first housing includes a first bearing part and a second bearing part, and the first positioning part is disposed between the first bearing part and the second bearing part.

The second housing is connected to the second elastic part. The second housing includes a third bearing part and a fourth bearing part, and the second positioning part is disposed between the third bearing part and the fourth bearing part.

The first bearing part and the third bearing part are arranged opposite to each other and form the receiving slot, and the second bearing part and the fourth bearing part are arranged opposite to each other and form the assembly slot.

In some embodiments, the first elastic part passes through the first bearing part and is provided with a first support structure, and the second elastic part passes through the second bearing part and is provided with a second support structure.

When the first elastic part is pressed, it is able to rotate around the first support structure to drive the first positioning part to move.

When the second elastic part is pressed, it can rotate around the second support structure to drive the second positioning part to move.

In some embodiments, the first elastic part includes a first pressing plate and a first connecting rod. The first pressing plate is disposed on the side of the first bearing part away from the first positioning part. The first connecting rod passes through the first bearing part and connects the first pressing plate and the first positioning part. The first pressing plate and/or the first connecting rod are connected to the first support structure.

The second elastic part includes a second pressing plate and a second connecting rod. The second pressing plate is disposed on the side of the third bearing part away from the second positioning part. The second connecting rod passes through the third bearing part and connects the second pressing plate and the second positioning part. The second pressing plate and/or the second connecting rod are connected to the second support structure.

When the first and second pressing plates are pressed, they are able to move closer to each other, causing the first connecting rod to rotate around the first support structure and the second connecting rod to rotate around the second support structure, thereby driving the first positioning part and the second positioning part to move away from each other.

In some embodiments, the end of the first elastic part away from the first positioning part is connected to the first housing, and the end of the second elastic part away from the second positioning part is connected to the second housing.

The first support structure and the second support structure are arranged opposite to each other, and when the first elastic part and the second elastic part are pressed, the first support structure and the second support structure come into contact with each other.

In some embodiments, the first elastic part is connected to the first housing via the first support structure, and the second elastic part is connected to the second housing via the second support structure.

In some embodiments, the limiting gap between the first positioning part and the second positioning part has a first dimension L1, the mating part has a second dimension L2, and the two dimensions satisfies: L1<L2.

In some embodiments, the receiving slot is provided with a limiting protrusion configured to limit the position of the clip assembly.

In a second aspect, the hemostatic clip kit described in the embodiments of the present disclosure includes:
a clip assembly;
a delivery device; and an assembly device as described in any of the above embodiments, wherein the assembly device is configured to assemble the clip assembly and the delivery device.

In a third aspect, an assembly method described in the embodiments of the present disclosure is provided, which is applied to the assembly device as described in any of the above embodiments, wherein the assembly method includes:
inserting the delivery device into the assembly slot through an opening of the housing assembly, and then into the receiving slot through the assembly slot, so that the positioning assembly of the assembly device engages with the mating part of the delivery device to position the delivery device, and the clip assembly is received in the receiving slot in a closed state;
manipulating the handle mechanism of the delivery device to insert a cable assembly of the delivery device into the clip seat of the clip assembly and connect it to the clip; and
completing the assembly by pulling the delivery device out of the assembly device and taking the clip assembly out with it.

The embodiments of the present disclosure have at least the following beneficial effects.

The assembly device according to the embodiments of the present disclosure has a housing assembly with a receiving slot and an assembly slot inside. The receiving slot is used to place the clip assembly, while the assembly slot is designed for the delivery device to be inserted through it into the receiving slot. Its positioning assembly is connected to the housing assembly and includes a first positioning part and a second positioning part disposed opposite to each other. The first positioning part and the second positioning part are disposed between the receiving slot and the assembly slot, and are configured to cooperate with a mating part when the delivery device is inserted into the receiving slot. With the above technical solution, during the assembly of the delivery device and the clip assembly, the delivery device can be inserted into the receiving slot via the assembly slot. When the delivery device is inserted into the receiving slot, the first positioning part and the second positioning part can cooperate with the mating part of the delivery device to position the delivery device, enabling it to accurately align with the clip assembly, thereby improving the efficiency of assembling the delivery device and the clip assembly and facilitating use.

The hemostatic clip kit according to the embodiments of the present disclosure includes the assembly device described above, and therefore can have all the technical features and effects of the assembly device, which will not be described in detail here.

The assembly method according to the embodiments of the present disclosure can improve the efficiency of assembling the delivery device and the clip assembly and facilitate use, and will not be described in detail here.

Other features and advantages of the present disclosure will be described in detail in the following detailed description of the embodiments.

### BRIEF DESCRIPTION OF THE DRAWINGS

To more clearly illustrate the technical solutions in the embodiments of the present disclosure, the accompanying drawings used in the description of the embodiments will be briefly introduced below. Obviously, the drawings described below are only some embodiments of the present disclosure. For those skilled in the art, other drawings can be obtained based on these drawings without creative effort.
FIG 1 is a schematic diagram of the overall structure of the assembly device provided in Embodiment 1 of the present disclosure.
FIG 2 is a schematic diagram of the exploded structure of the parts of the assembly device provided in Embodiment 1 of the present disclosure.
FIG 3 is a schematic diagram of the exploded structure of the parts in the state of assembling the clip assembly and the delivery device provided in Embodiment 1 of the present disclosure.
FIG 4 is a front view of the assembly device provided in Embodiment 1 of the present disclosure.
FIG 5 is a structural diagram of the assembly device provided in Embodiment 1 of the present disclosure after being cut along line A-A in FIG 4.
FIG 6 is a schematic diagram of the exploded structure of the parts of the assembly device provided in Embodiment 1 of the present disclosure from another perspective.
FIG 7 is a diagram showing the mating structure of the first housing and clip assembly of the assembly device provided in Embodiment 1 of the present disclosure.
FIG 8 is a partially enlarged structural diagram of region A in FIG 7.
FIG 9 is a schematic diagram of the overall structure of the assembly device provided in Embodiment 2 of the present disclosure.
FIG 10 is a schematic diagram of the exploded structure of the parts of the assembly device provided in Embodiment 2 of the present disclosure.
FIG 11 is a schematic diagram of the exploded structure of the parts in the state of assembling the clip assembly and the delivery device provided in Embodiment 2 of the present disclosure.
FIG 12 is a front view showing the mating structure of the first housing and the clip assembly of the assembly device, and the delivery device provided in Embodiment 2 of the present disclosure.
FIG 13 is a diagram showing the mating structure of the first housing and clip assembly of the assembly device provided in Embodiment 2 of the present disclosure.
FIG 14 is a partially enlarged structural diagram of region B in FIG 13.
FIG 15 is a front view of the assembly device provided in Embodiment 2 of the present disclosure in the state of assembling the clip assembly and the delivery device.
FIG 16 is a schematic diagram of the exploded structure of the part after being cut along line B-B in FIG 15.
FIG 17 is a schematic diagram of the exploded structure of the part from another perspective after being cut along line B-B in FIG 15.
FIG 18 is a schematic diagram of the structure after being cut along line O-O in FIG 15.
FIG 19 is a schematic diagram of the structure from another perspective after being cut along line O-O in FIG 15.
FIG 20 is an exploded view of the parts of another structure of the assembly device provided in Embodiment 2 of the present disclosure.
FIG 21 is a schematic diagram of the overall structure of the assembly device provided in Embodiment 3 of the present disclosure.
FIG 22 is a schematic diagram of the exploded structure of the parts of the assembly device provided in Embodiment 3 of the present disclosure.
FIG 23 is a schematic diagram of the exploded structure of the parts of the assembly device provided in Embodiment 3 of the present disclosure in the state of assembling the clip assembly and the delivery device.
FIG 24 is a front view showing the mating structure of the assembly device, the clip assembly, and the delivery device provided in Embodiment 3 of the present disclosure.
FIG 25 is a structural diagram showing the mating of the assembly device and clip assembly provided in Embodiment 3 of the present disclosure after being cut along line C-C in FIG 24.
FIG 26 is a partially enlarged structural diagram of region C in FIG 25.
FIG 27 is a schematic diagram of the structure after being cut along line C-C in FIG 24.
FIG 28 is a schematic diagram of the exploded structure of the parts of the assembly device provided in Embodiment 3 of the present disclosure after being cut along line C-C in FIG 24.
FIG 29 is a schematic diagram of the exploded structure of the parts of the assembly device provided in Embodiment 3 of the present disclosure from another perspective after being cut along line C-C in FIG 24.
FIG 30 is a schematic diagram of the overall structure of the assembly device provided in Embodiment 4 of the present disclosure.
FIG 31 is a schematic diagram of the exploded structure of the parts of the assembly device provided in Embodiment 4 of the present disclosure.
FIG 32 is a schematic diagram of the exploded structure of the parts of the delivery device provided in Embodiment 4 of the present disclosure in the state of assembling the clip assembly and the delivery device.
FIG 33 is a front view of the assembly device provided in Embodiment 4 of the present disclosure.
FIG 34 is a partially enlarged structural diagram of region D in FIG 33.
FIG 35 is a schematic diagram of the structure after being cut along line D-D in FIG 33.
FIG 36 is a schematic diagram of the assembled clip assembly and delivery device.
FIG 37 is a schematic diagram of the structure after being cut along line E-E in FIG 36.

List of reference numerals in the drawings:
10 - Assembly Device;
30 - Clip Assembly;
31 - Clip Seat;
311 - First Section;
312 - Second Section;
313 - Joint Part;
32 - Clip;
321 - Latch;
3211 - Retaining Unit;
3212 - Retaining Part;
322 - Clip Arm Element;
40 - Delivery Device;
41 - Sheath Assembly;
42 - Cable Assembly;
421 - Cable Body;
422 - Transmission Head;
43 - Connecting Assembly;
100 - Housing Assembly;
110 - First Housing;
120 - Second Housing;
121 - Pressing Part;
122 - Avoidance Part;
1210 - First Cutout;
1211 - Second Cutout;
123 - First Boss;
1231 - First Contact Surface;
124 - Second Boss;
1241 - Second Contact Surface;
130 - Receiving Slot;
140 - Assembly Slot;
141 - Guide Surface;
150 - First Bearing Part;
160 - Second Bearing Part;
170 - Third Bearing Part;
180 - Fourth Bearing Part;
190 - Limiting Protrusion;
200 - Positioning Assembly;
210 - First Positioning Part;
220 - Second Positioning Part;
230 - First Elastic Part;
231 - First Support Structure;
232 - First Pressing Plate;
233 - First Connecting Rod;
240 - Second Elastic Part;
241 - Second Support Structure;
242 - Second Pressing Plate;
243 - Second Connecting Rod;
250 - First Chamfered Bevel;
260 - Second Chamfered Bevel;
270 - Movement Clearance;
410 - Mating Part;
X - First Direction;
Y - Second Direction;
Z - Third Direction.

### DETAILED DESCRIPTION OF THE EMBODIMENTS

The technical solutions of the embodiments of the present disclosure will be clearly and completely described below with reference to the accompanying drawings. Obviously, the described embodiments are only a part of the embodiments of the present disclosure, and not all of them. All other embodiments obtained by those skilled in the art based on the embodiments of the present disclosure without creative effort are within the protection scope of the present disclosure.

In the description of the present disclosure, it should be understood that the terms "proximal end" and "distal end" are relative to the operator (doctor), where "proximal end" is the end closer to the operator and "distal end" is the end farther from the operator relative to "proximal end". The terms "upper," "lower," "inner," and "outer," etc., indicate the orientation or positional relationship based on the orientation or positional relationship shown in the accompanying drawings, and are only for the convenience of describing the present disclosure and simplifying the description, and do not indicate or imply that the device or apparatus referred to must have a specific orientation, or be configured and operated in a specific orientation, and therefore should not be construed as a limitation of the present disclosure. "A plurality" means two or more, and "at least one" can mean one, two, or more, unless otherwise explicitly specified. The terms "mounted," "connected," and "coupled" should be interpreted broadly, for example, they can refer to a fixed connection, a detachable connection, or an integral connection; they can refer to a direct connection or an indirect connection through an intermediate medium; they can refer to the internal communication of two devices or the interaction between two devices, unless otherwise explicitly specified. The terms "first" and "second" are used for descriptive purposes only and should not be construed as indicating or implying relative importance or implicitly specifying the number of indicated technical features. Therefore, a feature defined with "first" and "second" may explicitly or implicitly include one or more of that feature. In the description of the present disclosure, "multiple" means two or more, unless otherwise explicitly specified.

Embodiments of the present disclosure provide an assembly device 10 for assembling a clip assembly 30 and a delivery device 40. The assembly device 10 enables accurate alignment of the delivery device 40 and the clip assembly 30, improving assembly efficiency.

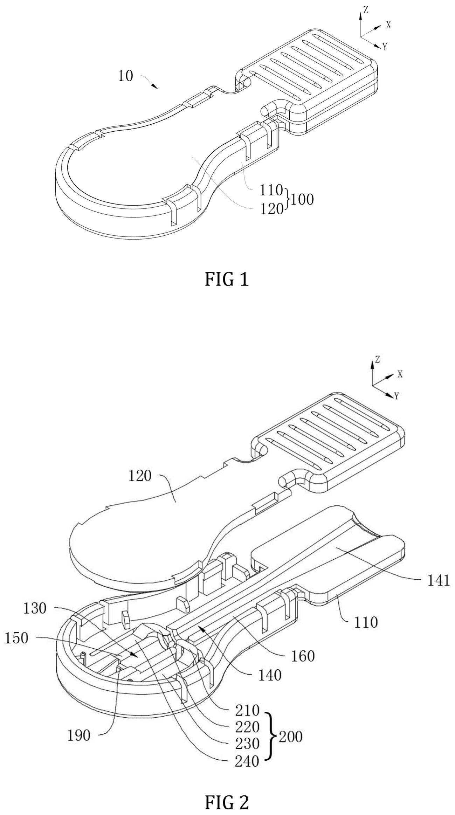

Referring to FIGS 1, 2, and 3 together, in the assembly device 10 provided in Embodiment 1 of the present disclosure, the housing assembly 100 includes a first housing 110 and a second housing 120. The first housing 110 and the second housing 120 are detachably connected by a snap-fit or slot-fitting method. In this embodiment, the housing assembly 100 is configured as two detachably connected parts, which facilitates opening the housing assembly 100 and placing the clip assembly 30 into the receiving slot 130 inside the housing assembly 100 for fixing. It is understood that the first housing 110 and the second housing 120 of the housing assembly 100 can also be connected by bolts, plug-in connections, or other methods. In addition, in some other embodiments, the housing assembly 100 can also be configured as an integral structure, that is, the housing assembly 100 is integrally formed and has an opening communicating with the internal receiving slot 130, through which the clip assembly 30 can be placed into the receiving slot 130.

Referring to FIG 2, the receiving slot 130 and an assembly slot 140 are formed inside the housing assembly 100. The receiving slot 130 is configured to place the clip assembly 30, and the assembly slot 140 is configured for the delivery device 40 to be inserted into the receiving slot 130. Specifically, the receiving slot 130 and the assembly slot 140 extend in the same direction, which is denoted as the first direction X. The first direction X is indicated by an arrow marked X in the figure. The first direction X may correspond to the length direction of the housing assembly 100. The receiving slot 130 and the assembly slot 140 are arranged sequentially along the first direction X.

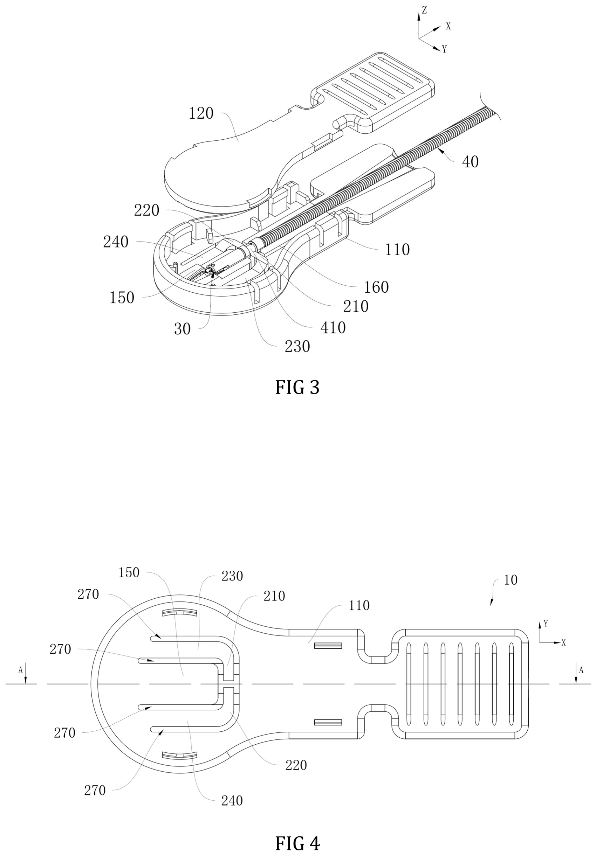

The housing assembly 100 is provided with an opening located on the side of the assembly slot 140 away from the receiving slot 130. Referring to FIG 3, the delivery device 40 may be inserted into the assembly slot 140 through the opening and inserted into the receiving slot 130 along the assembly slot 140 so as to be able to connect with the clip assembly 30.

The side wall of the assembly slot 140 near the opening is configured as a guide surface 141. The dimension of the guide surface 141 decreases from the opening inward and is roughly conical. The guide surface 141 may guide the delivery device 40 so that it can be accurately inserted into the assembly device 10.

The positioning assembly 200 is connected to the housing assembly 100, and includes a first positioning part 210 and a second positioning part 220 disposed opposite to each other, between the receiving slot 130 and the assembly slot 140. Referring to FIG 3, the first positioning part 210 and the second positioning part 220 are configured to cooperate with the mating part 410 of the delivery device 40 to position the delivery device 40 when the delivery device 40 is inserted into the receiving slot 130, so that the delivery device 40 can be accurately aligned with the clip assembly 30 for efficient assembly.

Specifically, referring again to FIG 2, the first housing 110 of the housing assembly 100 includes a first bearing part 150 and a second bearing part 160. The first bearing part 150 and the second bearing part 160 are protruding structures provided on the inner walls of the first housing 110, arranged sequentially along the first direction X and spaced apart from each other. The receiving slot 130 is a groove structure formed on the first bearing part 150, and the assembly slot 140 is a groove structure formed on the second bearing part 160. The first positioning part 210 and the second positioning part 220 are located between the first bearing part 150 and the second bearing part 160, and are arranged opposite to each other in the second direction Y. The first direction X and the second direction Y are perpendicular to each other. In the figures, the arrow marked Y indicates the second direction Y. In this embodiment, the second direction Y can correspond to the width direction of the housing assembly 100. It can be understood that the "opposite" in the context of the first positioning part 210 and the second positioning part 220 being arranged opposite to each other means that the first positioning part 210 and the second positioning part 220 are facing each other in space.

As shown in FIG 3, the clip assembly 30 may be placed in the receiving slot 130, with its clip seat 31 facing the side where the assembly slot 140 is located, to facilitate connection to the delivery device 40. Referring to FIG 2, the receiving slot 130 is also provided with a limiting protrusion 190, which is used to limit the position of the clip assembly 30, so that the clip assembly 30 can be stably placed in the receiving slot 130. When the clip assembly 30 is placed in the receiving slot 130, the limiting protrusion 190 abuts against the side of the clip seat 31 away from the second bearing part 160 to limit the position of the clip assembly 30.

The delivery device 40 passes through the gap between the first positioning part 210 and the second positioning part 220 via the assembly slot 140 and is inserted into the receiving slot 130. In this case, the first positioning part 210 and the second positioning part 220 may cooperate with the mating part 410 provided on the transition cap of the delivery device 40 to position the delivery device 40, so that the delivery device 40 can be quickly aligned with the clip assembly 30, thereby improving the efficiency of assembling the two members.

In Embodiment 1, the mating part 410 may be an annular groove on the transition cap of the delivery device 40, and the first positioning part 210 and the second positioning part 220 can be embedded in the annular groove to position the delivery device 40 in cooperation therewith. Alternatively, in other embodiments, the mating part 410 may be a protrusion (protruding outward) on the transition cap, and the first positioning part 210 and the second positioning part 220 may be configured as a groove structure that can mate with the protrusion, that is, the surfaces of the first positioning part 210 and the second positioning part 220 that contact the mating part 410 are set as grooves, and mating is achieved by embedding the protrusion into the groove. Alternatively, the mating part 410 may be a positioning hole on the transition cap, and the first positioning part 210 and the second positioning part 220 may be embedded in the positioning hole to achieve mating. Alternatively, the mating part 410 may be a notch formed on the surface of the transition cap, and the first positioning part 210 and the second positioning part 220 may be engaged into the notch to achieve mating.

Referring to FIGS 2 to 5, in Embodiment 1, the positioning assembly 200 further includes a first elastic part 230 and a second elastic part 240. The first positioning part 210 is connected to the housing assembly 100 through the first elastic part 230, and the second positioning part 220 is connected to the housing assembly 100 through the second elastic part 240. The first elastic part 230 and the second elastic part 240 are configured to undergo elastic deformation. When the positioning assembly 200 is subjected to pressure, the first elastic part 230 and the second elastic part 240 undergo elastic deformation, allowing the first positioning part 210 and the second positioning part 220 to move.

During the process of inserting the delivery device 40 into the receiving slot 130 via the assembly slot 140, the transition cap of the delivery device 40 contacts and presses the first positioning part 210 and the second positioning part 220. The positioning assembly 200 is subjected to the pressing force from the delivery device 40, which is transmitted to the first elastic part 230 and the second elastic part 240, causing them to elastically deform to both sides in the second direction Y. This, in turn, causes the first positioning part 210 and the second positioning part 220 to move away from each other in the second direction Y, allowing the delivery device 40 to smoothly pass through the first positioning part 210 and the second positioning part 220 and be inserted into the receiving slot 130. As the delivery device 40 continues to move, the first positioning part 210 and the second positioning part 220 cooperate with the mating part 410 to position the delivery device 40, enabling the delivery device 40 to be assembled with the clip assembly 30.

Referring to FIG 4, in Embodiment 1, the first elastic part 230 and the second elastic part 240 are respectively disposed on both sides of the first bearing part 150 in the second direction Y. In the second direction Y, both sides of the first elastic part 230 and the second elastic part 240 have movement clearances 270, allowing the first elastic part 230 and the second elastic part 240 to deform towards their respective movement clearances 270. The transition cap of the delivery device 40 contacts and presses the first positioning part 210 and the second positioning part 220, causing the first elastic part 230 and the second elastic part 240 to elastically deform towards the movement clearances 270, thereby driving the first positioning part 210 and the second positioning part 220 away from each other in the second direction Y.

When the first positioning part 210 and the second positioning part 220 are engaged with the mating part 410, due to the elastic recovery ability of the first elastic part 230 and the second elastic part 240, the first positioning part 210 and the second positioning part 220 may press the mating part 410, thereby improving the stability of the engagement between the first positioning part 210 and the second positioning part 220 and the mating part 410.

Referring to FIGS 4, 5, and 6 together, in Embodiment 1, the positioning assembly 200 and the first housing 110 are an integral structure, that is, the first positioning part 210, the second positioning part 220, the first elastic part 230, the second elastic part 240, and the first housing 110 are a single unit, which can be integrally formed by injection molding, 3D printing, or other processing methods. For an integral structure, structural stability can be effectively improved and processing difficulty reduced. Furthermore, for an integral structure, the movement clearance 270 on both sides of the first elastic part 230 and the second elastic part 240 can reduce the rigidity of the structure at that location, improve the elastic deformation capability of the first elastic part 230 and the second elastic part 240, and facilitate the insertion of the delivery device 40.

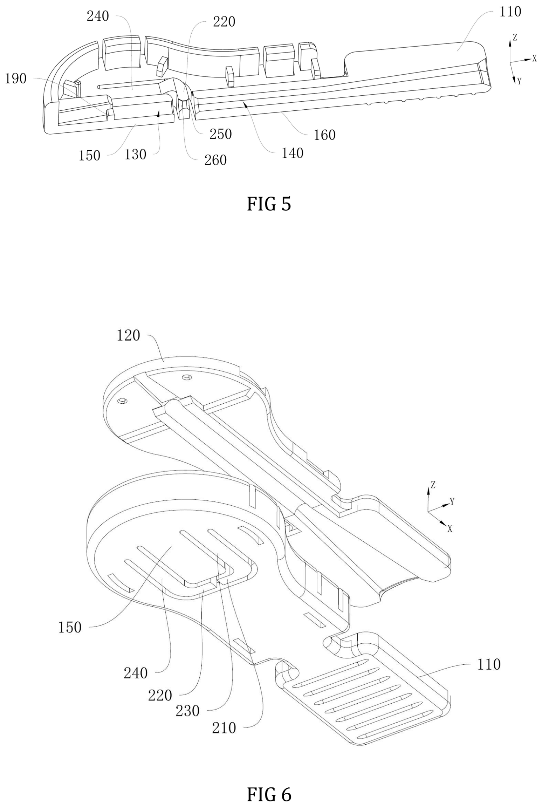

Referring to FIG 6 again, in Embodiment 1, the inner wall of the second housing 120 facing the first housing 110 is provided with a groove structure opposite to the receiving slot 130 and the assembly slot 140. This groove structure may cooperate with the receiving slot 130 and the assembly slot 140, so that the clip assembly 30 can be placed more stably in the housing assembly 100 and can guide the delivery device 40 more stably.

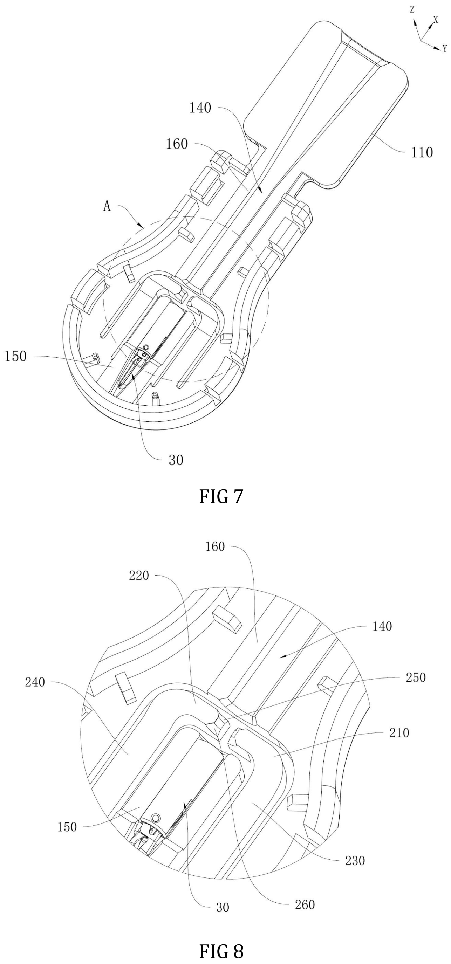

Referring to FIGs 7 and 8 together, in Embodiment 1, the first positioning part 210 and/or the second positioning part 220 have a first chamfered bevel 250 on the side near the assembly slot 140. When the delivery device 40 moves from the assembly slot 140 to the receiving slot 130, the first chamfered bevel 250 can contact the delivery device 40. By providing the first chamfered bevel 250 on the side of the first positioning part 210 and/or the second positioning part 220 facing the assembly slot 140, on the one hand, the first chamfered bevel 250 can be used to guide the delivery device 40, so that the delivery device 40 can be inserted into the receiving slot 130 more accurately; on the other hand, the delivery device 40 pressing against the first chamfered bevel 250 also makes it easier to move the first positioning part 210 and the second positioning part 220 away from each other, so that the delivery device 40 can be inserted into the receiving slot 130 more smoothly. The operator can directly insert the delivery device 40 into the receiving slot 130 and connect it with the clip assembly 30, thereby improving the accuracy and efficiency of assembling the delivery device 40 and the clip assembly 30, and facilitating operation by medical personnel. Optionally, both the first positioning part 210 and the second positioning part 220 have a first chamfered bevel 250.

Referring again to FIG 8, the first positioning part 210 and/or the second positioning part 220 have a second chamfered bevel 260 on the side near the receiving slot 130. When the delivery device 40 moves from the receiving slot 130 toward the assembly slot 140, the second chamfered bevel 260 can contact the delivery device 40. By providing the second chamfered bevel 260 on the side of the first positioning part 210 and/or the second positioning part 220 facing the receiving slot 130, after the delivery device 40 and the clip assembly 30 are connected, the connected delivery device 40 and clip assembly 30 can be directly pulled out from the assembly device 10, reducing the resistance of the first positioning part 210 and the second positioning part 220. Optionally, both the first positioning part 210 and the second positioning part 220 have the second chamfered bevel 260.

The first chamfered bevel 250 and the second chamfered bevel 260 facilitate the insertion or removal of the delivery device 40 from the assembly device 10. According to actual clinical needs, after setting the first chamfered bevel 250 and the second chamfered bevel 260, the force required for the delivery device 40 to be inserted or removed may not exceed 50N.

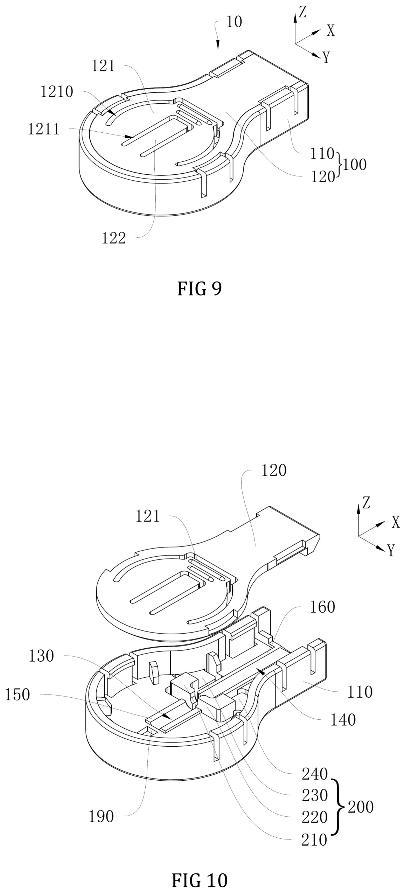

Referring to FIGS 9, 10, and 11 together, the structure of Embodiment 2 of the present disclosure is similar to that of Embodiment 1. In Embodiment 2, the housing assembly 100 is also configured as two parts: a first housing 110 and a second housing 120, which are detachably connected by a snap-fit or slot-fitting method. A first bearing part 150 and a second bearing part 160 are arranged along the first direction X and spaced apart from each other on the inner wall of the first housing 110. A receiving slot 130 is formed on the first bearing part 150, and an assembly slot 140 is formed on the second bearing part 160. A first positioning part 210 and a second positioning part 220 are disposed between the first bearing part 150 and the second bearing part 160, and are positioned opposite each other along the second direction Y.

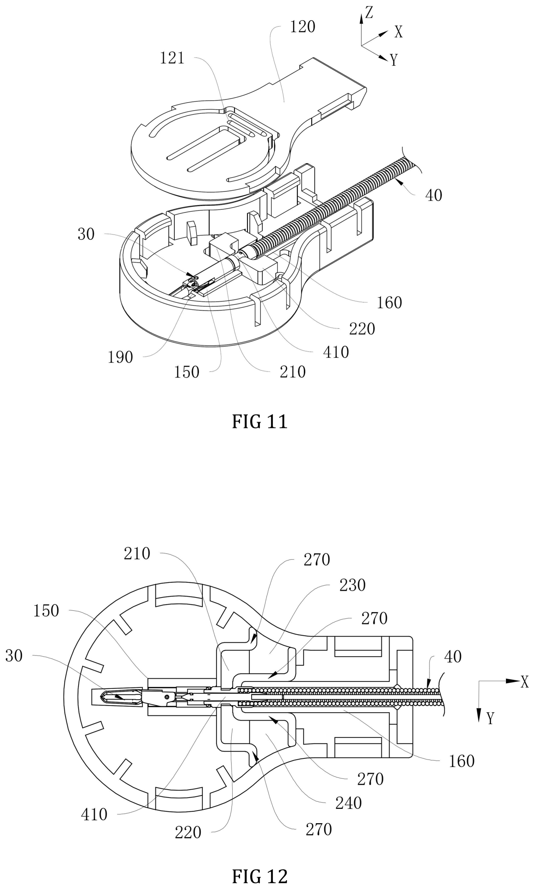

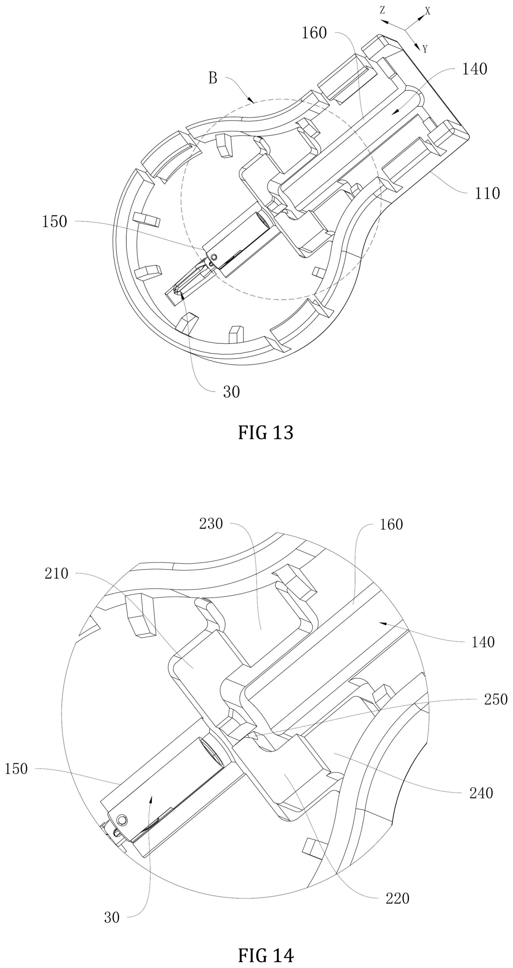

Referring to FIGS 12 and 13, in Embodiment 2, the positions of the first elastic part 230 and the second elastic part 240 are slightly different from those in Embodiment 1. They are respectively disposed on both sides of the second bearing part 160 in the second direction Y. The first elastic part 230 and the second elastic part 240 are respectively connected to the side walls of the first housing 110, and both have a movement clearance 270 between themselves and the first bearing part 150 and the second bearing part 160.

Referring to FIGS 13 and 14 together, in Embodiment 2, the first positioning part 210 and the second positioning part 220 have a first chamfered bevel 250 on the side near the assembly slot 140. This allows the operator to directly insert the delivery device 40 into the receiving slot 130 and connect it with the clip assembly 30, thereby improving the accuracy and efficiency of assembling the delivery device 40 and the clip assembly 30, and facilitating operation by medical personnel.

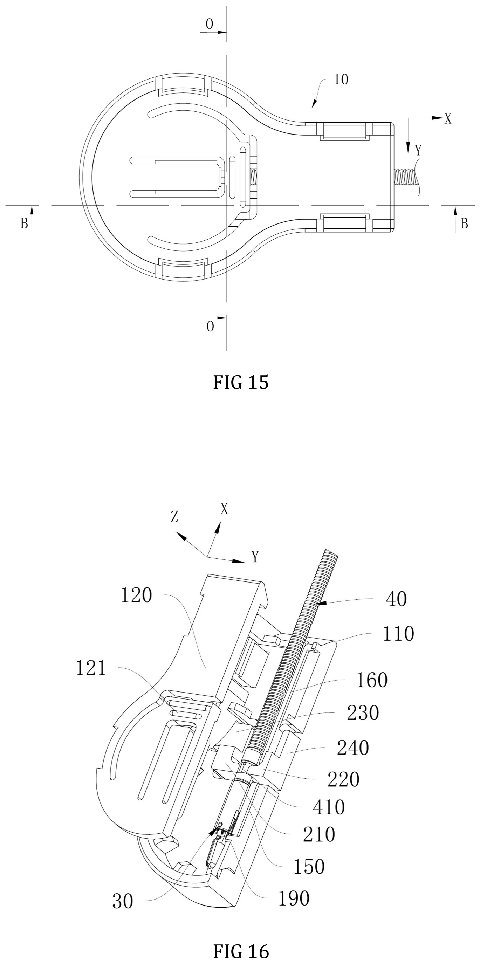

Referring to FIGS 15 to 19, unlike Embodiment 1, in Embodiment 2, the second housing 120 includes a pressing part 121. The pressing part 121 can apply pressure to the positioning assembly 200, causing the first positioning part 210 and the second positioning part 220 to move away from each other. In this embodiment, the second housing 120 and the positioning assembly 200 are positioned opposite each other in the third direction Z. The first direction X, the second direction Y, and the third direction Z are perpendicular to each other. The arrow marked Z in the figures indicates the third direction Z, which can correspond to the thickness direction of the housing assembly 100. When pressed, the pressing part 121 of the second housing 120 can move towards the positioning assembly 200, contacting the positioning assembly 200 and applying pressure to it, causing the first positioning part 210 and the second positioning part 220 to separate from each other in the second direction Y, thereby detaching them from the mating part 410. This allows the connected delivery device 40 and clip assembly 30 to be smoothly pulled out of the assembly device 10, facilitating assembly operations.

Specifically, referring again to FIG 9, a first cutout 1210 and a second cutout 1211 are formed on the second housing 120. The first cutout 1210 and the second cutout 1211 change the structural rigidity between them, so that a movable pressing part 121 is formed between them. The pressing part 121 can undergo elastic deformation when pressed. The second housing 120 also has an avoidance part 122, which is disposed inside the pressing part 121, and the second cutout 1211 is formed between them. The avoidance part 122 is disposed opposite to the first bearing part 150 in the third direction Z. The side of the avoidance part 122 facing the first bearing part 150 has a groove structure. The groove structure is opposite to and cooperates with the receiving slot 130, so that the clip assembly 30 is placed more stably. By providing the avoidance part 122, and by providing the second groove 1211 to separate the connection between the avoidance part 122 and the pressing part 121, the pressing part 121 can avoid the clip assembly 30 when it is pressed and displaced, thus preventing the clip assembly 30 from being squeezed.

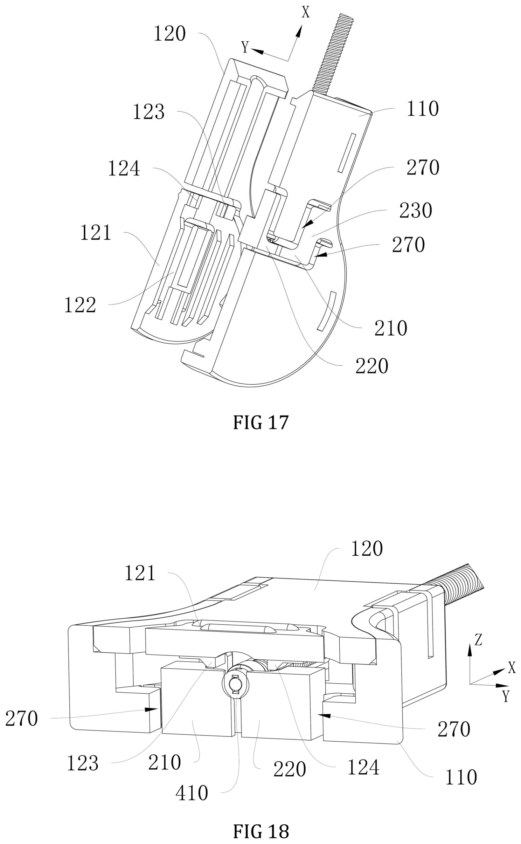

Referring to FIGS 17, 18, and 19 together, the pressing part 121 is provided with a first boss 123 and a second boss 124 protruding toward the positioning assembly 200. When the pressing part 121 moves toward the positioning assembly 200, the first boss 123 and the second boss 124 can move toward the first positioning part 210 and the second positioning part 220, and the first boss 123 can press the first positioning part 210, and the second boss 124 can press the second positioning part 220, so that the first positioning part 210 and the second positioning part 220 can separate from each other in the second direction Y.

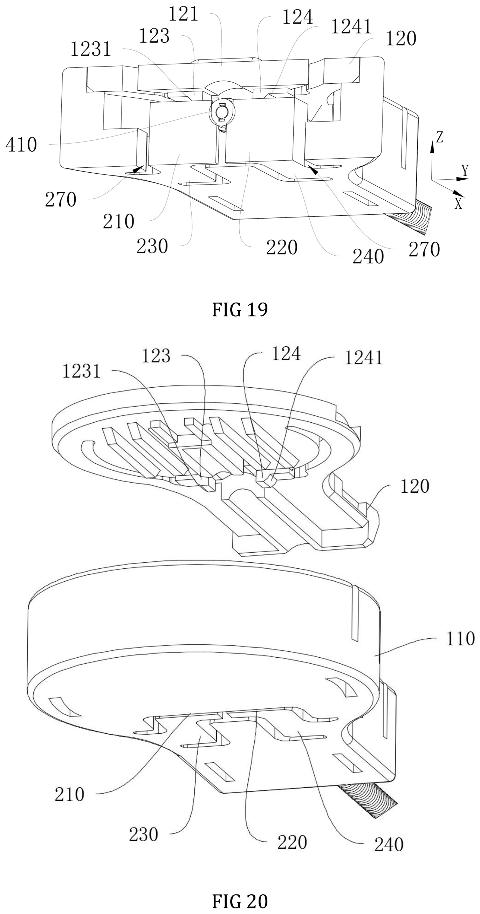

The first boss 123 has a first contact surface 1231 for contacting and pressing the first positioning part 210; the second boss 124 has a second contact surface 1241 for contacting and pressing the second positioning part 220. As shown in FIG19, both the first contact surface 1231 and the second contact surface 1241 are inclined surfaces. As shown in FIG 20, both the first contact surface 1231 and the second contact surface 1241 are arc surfaces. By setting the contact surface between the first boss 123 and the first positioning part 210, and the contact surface between the second boss 124 and the second positioning part 220 as inclined or arc surfaces, as the pressing depth increases, the pressing amplitude of the first contact surface 1231 on the first positioning part 210 and the second contact surface 1241 on the second positioning part 220 increases, so that the first positioning part 210 and the second positioning part 220 can move away from each other.

Referring to FIG 14 again, at least a portion of the first positioning part 210 protrudes toward the pressing part 121 relative to the first elastic part 230, and at least a portion of the second positioning part 220 protrudes toward the pressing part 121 relative to the second elastic part 240, so that the pressing part 121 may apply a pressing force to the first positioning part 210 and the second positioning part 220.

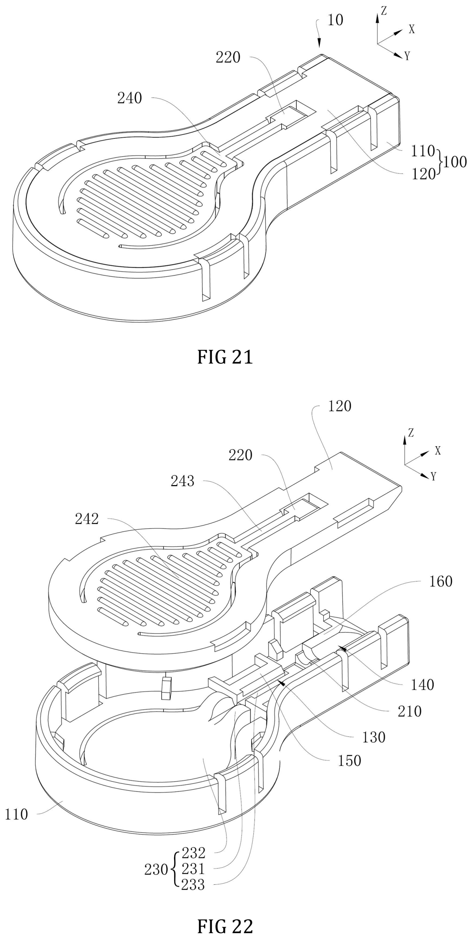

Referring to FIGS 21, 22 and 23 together, in Embodiment 3 of the present disclosure, the first elastic part 230 and the first positioning part 210 are disposed on the first housing 110, and the second elastic part 240 and the second positioning part 220 are disposed on the second housing 120.

Specifically, the first housing 110 is connected to the first elastic part 230. The first housing 110 includes a first bearing part 150 and a second bearing part 160, which are arranged in a first direction X and spaced apart from each other. The first positioning part 210 is disposed between the first bearing part 150 and the second bearing part 160. The first elastic part 230 passes through the first bearing part 150 in the first direction X and is connected to the first positioning part 210.

The second housing 120 is connected to the second elastic part 240. The second housing 120 includes a third bearing part 170 and a fourth bearing part 180, which are arranged in a first direction X and spaced apart from each other. The second positioning part 220 is disposed between the third bearing part 170 and the fourth bearing part 180. The second elastic part 240 passes through the third bearing part 170 in the first direction X and is connected to the second positioning part 220.

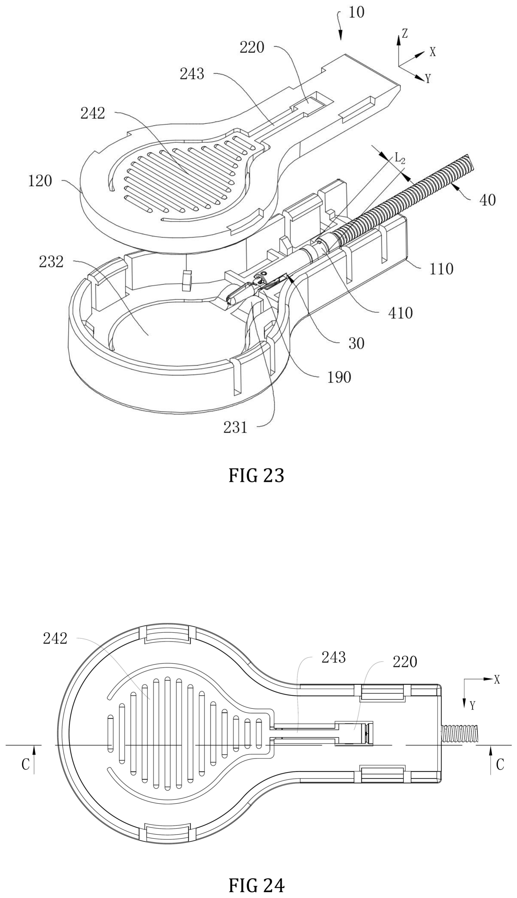

Referring to FIGS 24 to 26 together, in Embodiment 3, the first bearing part 150 and the third bearing part 170 are arranged opposite each other and form the receiving slot 130. The clip assembly 30 is placed in the receiving slot 130 between the first bearing part 150 and the third bearing part 170 and is fixed by the limiting protrusion 190 shown in FIG 23. The second bearing part 160 and the fourth bearing part 180 are arranged opposite each other and form the assembly slot 140. The delivery device 40 can be inserted from the assembly slot 140 between the second bearing part 160 and the fourth bearing part 180 into the receiving slot 130 to connect with the clip assembly 30.

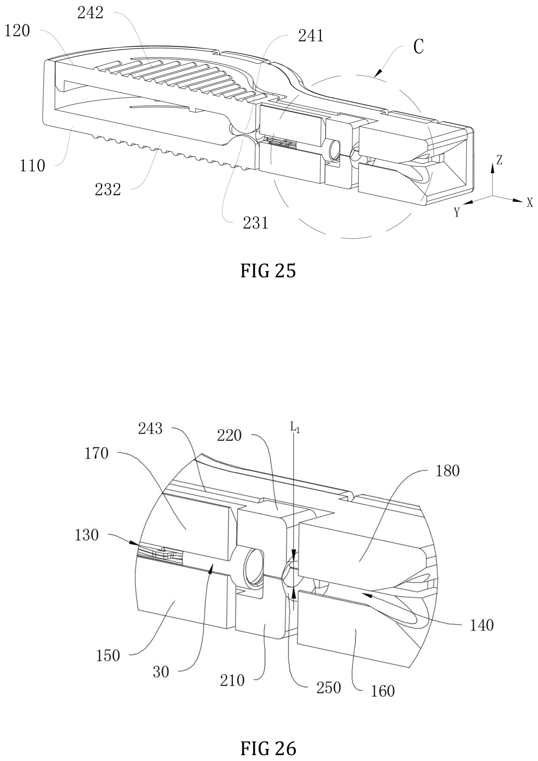

As shown in FIG 26, during the process of the delivery device 40 being inserted into the receiving slot 130 via the assembly slot 140, the delivery device 40 will pass through the gap between the first positioning part 210 and the second positioning part 220. The first positioning part 210 and the second positioning part 220 can cooperate with the mating part 410 to position the delivery device 40.

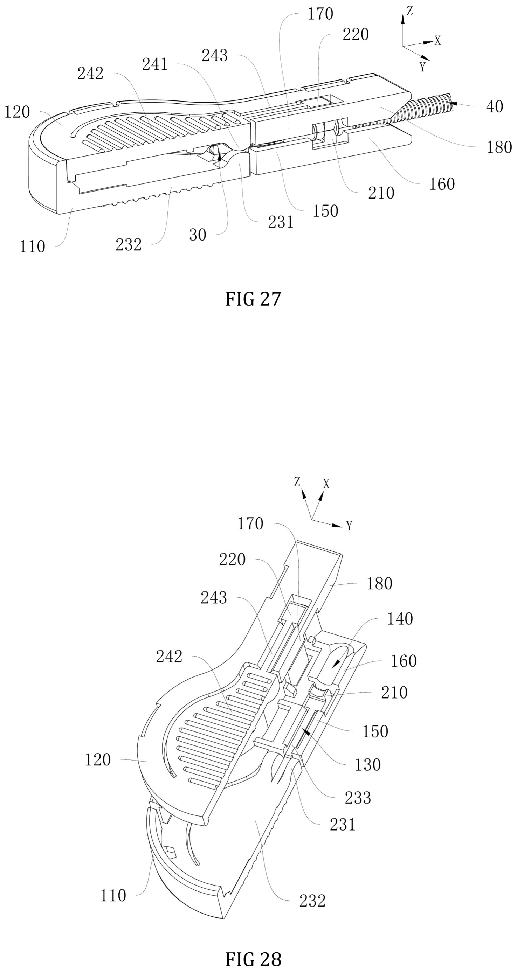

Referring to FIGS 27, 28, and 29 together, in Embodiment 3, the first elastic part 230 is provided with a first support structure 231. When pressed, the first elastic part 230 may rotate around the first support structure 231 to drive the first positioning part 210 to move. The second elastic part 240 is provided with a second support structure 241. When pressed, the second elastic part 240 may rotate around the second support structure 241 to drive the second positioning part 220 to move.

Specifically, the end of the first elastic part 230 away from the first positioning part 210 is connected to the first housing 110, and the end of the second elastic part 240 away from the second positioning part 220 is connected to the second housing 120. The first support structure 231 is a protrusion of the first elastic part 230 facing the second elastic part 240, and the second support structure 241 is a protrusion of the second elastic part 240 facing the first elastic part 231. The first support structure 231 and the second support structure 241 are arranged opposite each other in the third direction Z. When the first elastic part 230 and the second elastic part 240 are pressed, the first support structure 231 and the second support structure 241 come into contact with each other. Thus, the first support structure 231 and the second support structure 241 support each other, forming a fulcrum. The first elastic part 230 and the second elastic part 240 can rotate around the fulcrum respectively, thereby driving the first positioning part 210 and the second positioning part 220 to move respectively, causing them to separate in the third direction Z, and thus disengaging from the mating part 410. This allows the connected delivery device 40 and clip assembly 30 to be easily removed from the assembly device 10.

As shown in FIG 28, in Embodiment 3, the first elastic part 230 includes a first pressing plate 232 and a first connecting rod 233. The first pressing plate 232 is configured to be pressed by a finger and is located on the side of the first bearing part 150 away from the first positioning part 210, with its end away from the first bearing part 150 connected to the first housing 110. In the first direction X, the first pressing plate 232, the first connecting rod 233, and the first positioning part 210 are arranged sequentially, and the first connecting rod 233 connects the first pressing plate 232 and the first positioning part 210. Specifically, the first connecting rod 233 passes through the first bearing part 150 in the first direction X to connect the first pressing plate 232 and the first positioning part 210 located on both sides of the first bearing part 150.

It is understood that in this embodiment, the first bearing part 150 includes two portions spaced apart from each other in the second direction Y. The two portions are respectively provided with groove structures, which, together with the groove structure of the third bearing part 170, form the receiving slot 130. The first connecting rod 233 is disposed between the two portions and passes through the first bearing part 150 in the first direction X.

In Embodiment 3, the first support structure 231 is disposed on the first pressing plate 232. Specifically, the first support structure 231 is disposed at the connection point between the first pressing plate 232 and the first connecting rod 233, and protrudes toward the second elastic part 240.

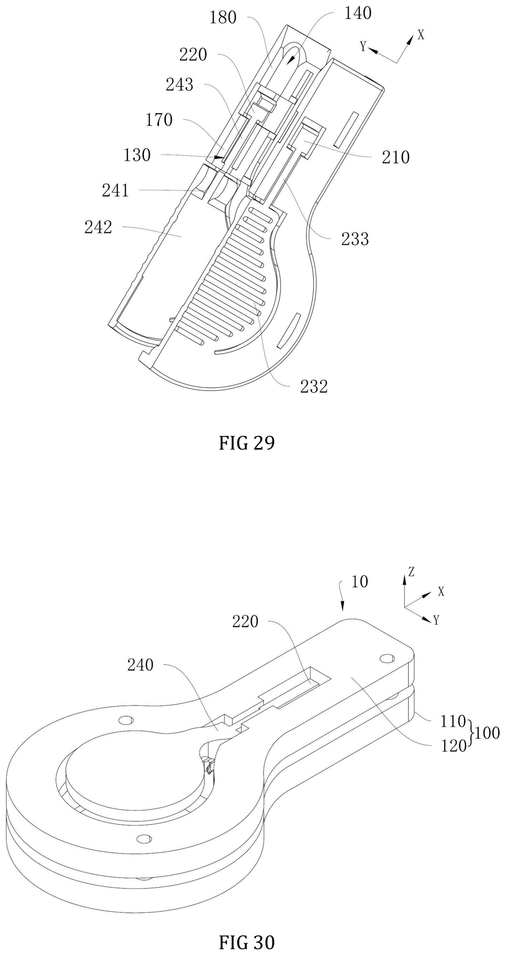

As shown in FIG 29, in Embodiment 3, the second elastic part 240 includes a second pressing plate 242 and a second connecting rod 243. The second pressing plate 242 is configured to be pressed by a finger and is located on the side of the third bearing part 170 away from the second positioning part 220, with its end away from the third bearing part 170 connected to the second housing 120. In the first direction X, the second pressing plate 242, the second connecting rod 243, and the second positioning part 220 are arranged sequentially, and the second connecting rod 243 connects the second pressing plate 242 and the second positioning part 220. Specifically, the second connecting rod 243 passes through the third bearing part 170 in the first direction X to connect the second pressing plate 242 and the second positioning part 220 located on both sides of the third bearing part 170.

It is understood that in this embodiment, the third bearing part 170 also includes two portions spaced apart from each other in the second direction Y. The two portions are respectively provided with groove structures, which, together with the groove structure of the first bearing part 150, form the receiving slot 130. The second connecting rod 243 is disposed between the two portions and passes through the third bearing part 170 in the first direction X.

In Embodiment 3, the second support structure 241 is disposed on the second pressing plate 242. Specifically, the second support structure 241 is disposed at the connection point between the second pressing plate 242 and the second connecting rod 243, and protrudes toward the first elastic part 230.

After the clip assembly 30 and the delivery device 40 are assembled, pressing the first pressing plate 232 and the second pressing plate 242 with a finger causes the first pressing plate 232 and the second pressing plate 242 to move closer together, causing the first connecting rod 233 to rotate around the first support structure 231 and the second connecting rod 243 to rotate around the second support structure 241. This causes the first positioning part 210 and the second positioning part 220 to move away from each other, thereby disengaging the first positioning part 210 and the second positioning part 220 from the mating part 410. In this way, the connected delivery device 40 and clip assembly 30 can be easily pulled out of the assembly device 10.

Referring again to FIGS 23 and 26, there is a first dimension L1 between the first positioning part 210 and the second positioning part 220, and the mating part 410 has a second dimension L2, and the two dimensions satisfies: L1<L2. Specifically, the limiting gap formed between the first positioning part 210 and the second positioning part 220 is a near-circular channel structure, and its minimum inner diameter is defined as the first dimension L1. The mating part 410 is correspondingly set to be approximately cylindrical, and its measured outer diameter in the same direction is the second dimension L2. When the mating part 410 is inserted into the limiting gap along the first direction X, due to the dimension setting L2>L1, the outer surface of the mating part 410 will create an interference fit with the contact surfaces of the first positioning part 210 and the second positioning part 220. At this time, the first positioning part 210 and the second positioning part 220 expand outward through elastic deformation, and the rebound force generated by the expansion forms a radial clamping force. This clamping force, together with the frictional force in the insertion direction, achieves reliable locking of the delivery device 40.

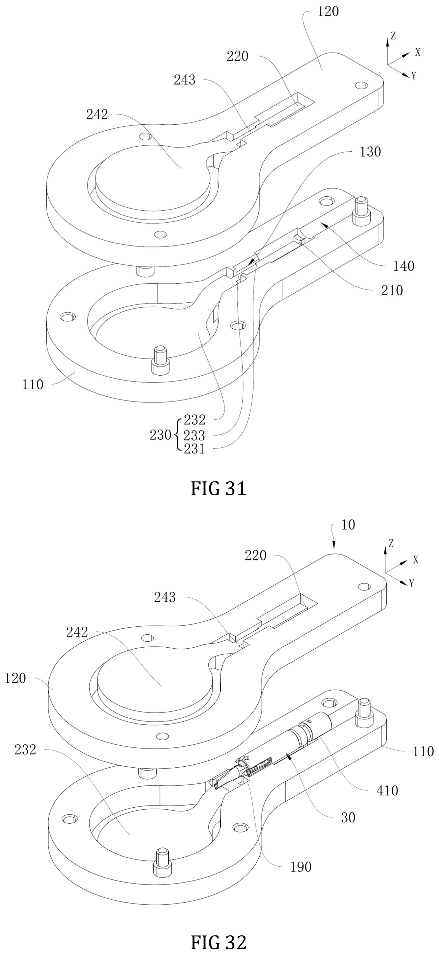

Referring to FIGS 30 to 35, the structure of Embodiment 4 is substantially the same as that of Embodiment 3, except that in Embodiment 4, the first elastic part 230 is connected to the first housing 110 via the first support structure 231, and the second elastic part 240 is connected to the second housing 120 via the second support structure 241. That is, in Embodiment 4, the first support structure 231 and the second support structure 241 are no longer configured as mutually cooperating protruding structures. Instead, the connection point between the first elastic part 230 and the first housing 110 serves as the first support structure 231, and the connection point between the second elastic part 240 and the second housing 120 serves as the second support structure 241. Using the connection point as a fulcrum, when the first elastic part 230 and the second elastic part 240 are pressed, they can rotate around the fulcrum formed by the connection point, that is, rotate around the first support structure 231 and the second support structure 241 respectively, thereby driving the first positioning part 210 and the second positioning part 220 to move.

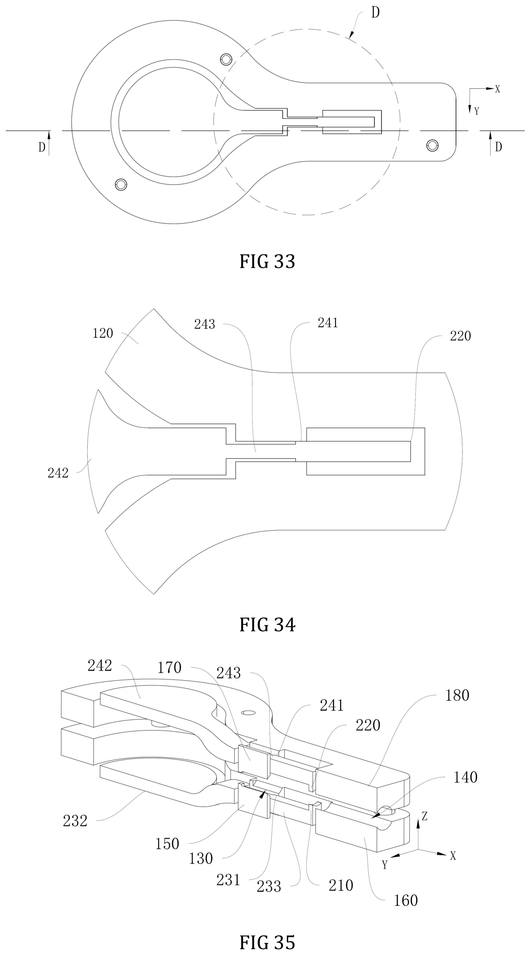

As shown in FIG 34, the two sides of the second connecting rod 243 are connected to the second housing 120 (specifically, connected to the two portions of the third bearing part 170 mentioned above), and the connection point is the second support structure 241. Correspondingly, the two sides of the first connecting rod 233 are connected to the first housing 110 (specifically, connected to the two portions of the first bearing part 150 mentioned above), and the connection point is the first support structure 231.

The present disclosure also provides a hemostatic clip kit, which includes a clip assembly 30, a delivery device 40, and the assembly device 10 as described in any of the above embodiments. The assembly device 10 is configured to assemble the clip assembly 30 and the delivery device 40. This hemostatic clip kit may have all the technical features and effects of the assembly device 10 described above, which will not be repeated here.

The clip assembly 30, the delivery device 40, and the assembly device 10 may be stored and transported independently, or the clip assembly 30 may be placed in the receiving slot 130 of the assembly device 10.

The present disclosure also provides an assembly method for the assembly device 10 of any of the above embodiments, as shown in FIG 1 and in conjunction with FIGS 11, 12, 36 and 37. The assembly method includes: inserting the delivery device 40 into the assembly slot 140 through the opening of the housing assembly 100, and then into the receiving slot 130 through the assembly slot 140, so that the positioning assembly 200 of the assembly device 10 engages with the mating part 410 of the delivery device 40 to position the delivery device 40, and the clip assembly 30 is received in the receiving slot 130 in a closed state; manipulating the handle mechanism of the delivery device 40 to insert the cable assembly 42 of the delivery device 40 into the clip seat 31 of the clip assembly 30 and connect it with the clip 32; and completing the assembly by pulling the delivery device 40 out of the assembly device 10 and taking the clip assembly 30 out with it.

Specifically, the sheath assembly 41 and the cable assembly 42 of the delivery device 40 are inserted together into the assembly slot 140 through the opening of the housing assembly 100, and then into the receiving slot 130 through the assembly slot 140. This allows the first positioning part 210 and the second positioning part 220 of the assembly device 10 to engage with the mating part 410 of the sheath assembly 41, thus fixing the distal transition cap of the sheath assembly 41. By manipulating the handle mechanism of the delivery device 40, the cable body 421 of the cable assembly 42, together with the transmission head 422, is moved toward the distal end, and the transmission head 422 is inserted into the clip seat 31 and connected to the clip 32. Here, the closed state refers to the clip arm elements 322 of the clip assembly 30 being in a mutually clamped state.

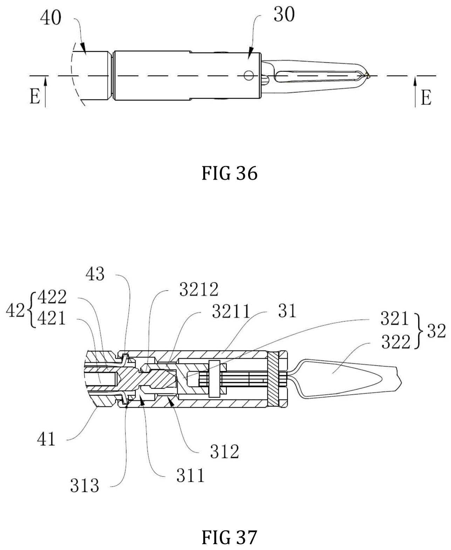

As shown in FIG 37, in some embodiments, the internal space of the clip seat 31 has a first section 311 and a second section 312 arranged sequentially along its axial direction. The first section 311 is located on the side of the second section 312 away from the clip arm element 322, and the radial dimension of the first section 311 is greater than the radial dimension of the second section 312. Here, "radial dimension" refers to the dimension of the inner contour of the structure along a direction perpendicular to the central axis of the clip assembly 30, and this radial dimension is independent of the cross-sectional shape (such as circular, polygonal, or irregular shape) of the clip assembly 30, and is based solely on its central axis. For example, if the internal space is cylindrical, the radial dimension is the length in the diameter direction; if the internal space is polygonal, the radial dimension is twice the vertical distance from the central axis to either sidewall.

When the clip assembly 30 is housed in the receiving slot 130 in the closed state as shown in FIGS 36 and 37, the retaining part 3212 of the retaining unit 3211 of the latch 321 of the clip 32 is located in the first section 311, so that the two sides of the retaining part 3212 are not constrained by the clip seat 31. During the insertion of the transmission head 422 into the latch 321, the retaining parts 3212 are pressed and can move away from each other, and the retaining unit 3211 undergoes elastic deformation. Therefore, the cone head of the transmission head 422 can be inserted between the retaining units 3211 to achieve connection with the latch 321. Furthermore, the cone head of the transmission head 422 is designed with a conical structure. When the transmission head 422 is inserted into the latch 321, the conical surface of the cone head contacts the latch 321 first, which can play a guiding role. Therefore, the force required to insert the latch 321 is very small, and it is relatively easy to insert into the latch 321. When the transmission head 422 is pulled out of the latch 321, there is no tapered guide, so the pulling force is large, which ensures that the clip arm element 322 enters the locking state when the tissue is clamped.

In the above embodiments, the descriptions of each embodiment have different focuses. For parts not described in detail in a certain embodiment, please refer to the relevant descriptions in other embodiments.

The assembly device 10 and the hemostatic clip kit provided in the embodiments of the present disclosure have been described in detail above, and specific examples have been used to illustrate the principles and implementation methods of the present disclosure. The description of the above embodiments is only for the purpose of helping to understand the technical solutions and core ideas of the present disclosure. Those skilled in the art should understand that they can still modify the technical solutions described in the foregoing embodiments, or make equivalent substitutions for some of the technical features; and these modifications or substitutions do not cause the essence of the corresponding technical solutions to deviate from the scope of the technical solutions of the embodiments of the present disclosure.

## Claims

1. An assembly device for assembling a clip assembly and a delivery device provided with a mating part, wherein the assembly device comprises:
a housing assembly, internally defining a receiving slot and an assembly slot, wherein the receiving slot is configured to accommodate the clip assembly, and the assembly slot is configured to allow the delivery device to be inserted into the receiving slot via the assembly slot; and
a positioning assembly connected to the housing assembly, comprising a first positioning part and a second positioning part arranged opposite to each other, wherein the first positioning part and the second positioning part are disposed between the receiving slot and the assembly slot, and are configured to engage with the mating part when the delivery device is inserted into the receiving slot.

2. The assembly device according to claim 1, wherein the positioning assembly further comprises a first elastic part and a second elastic part, the first positioning part being connected to the housing assembly through the first elastic part, and the second positioning part being connected to the housing assembly through the second elastic part; and
when the positioning assembly is subjected to pressure, the first elastic part and the second elastic part undergo elastic deformation, so that the first positioning part and the second positioning part are able to move.

3. The assembly device according to claim 1, wherein the first positioning part and/or the second positioning part have a first chamfered bevel on the side near the assembly slot; and
when the delivery device moves from the assembly slot to the receiving slot, the first chamfered bevel is able to contact the delivery device.

4. The assembly device according to claim 1, wherein the first positioning part and/or the second positioning part have a second chamfered bevel on the side near the receiving slot; and
when the delivery device moves from the receiving slot toward the assembly slot, the second chamfered bevel is able to contact the delivery device.

5. The assembly device according to claim 2, wherein the housing assembly comprises a first housing and a second housing connected to the first housing.

6. The assembly device according to claim 5, wherein the first housing includes a first bearing part and a second bearing part, the receiving slot is disposed in the first bearing part, the assembly slot is disposed in the second bearing part, and the first positioning part and the second positioning part are disposed between the first bearing part and the second bearing part.

7. The assembly device according to claim 6, wherein the arrangement direction of the first bearing part and the second bearing part is a first direction, the first positioning part and the second positioning part are disposed opposite to each other in a second direction, and the first direction and the second direction are perpendicular to each other;
the first elastic part and the second elastic part are respectively disposed on both sides of the first bearing part in the second direction, or the first elastic part and the second elastic part are respectively disposed on both sides of the second bearing part in the second direction; and
both sides of the first elastic part and the second elastic part have movement clearances.

8. The assembly device according to claim 7, wherein, when the positioning assembly is subjected to pressure exerted by the delivery device, the first positioning part and the second positioning part are able to move away from each other in the second direction.

9. The assembly device according to claim 7, wherein the second housing and the positioning assembly are disposed opposite each other in a third direction, and the first direction, the second direction, and the third direction are perpendicular to each other; and
the second housing comprises a pressing part, which, pressed, is able to move toward the positioning assembly and apply pressure to the positioning assembly, so that the first positioning part and the second positioning part move away from each other in the second direction.

10. The assembly device according to claim 9, wherein the pressing part is provided with a first boss and a second boss facing the positioning assembly; and
when the pressing part moves toward the positioning assembly, the first boss presses the first positioning part, and the second boss presses the second positioning part, so that the first positioning part and the second positioning part are able to move away from each other in the second direction.

11. The assembly device according to claim 10, wherein the first boss has a first contact surface for contacting and pressing the first positioning part, the first contact surface being an inclined surface or an arc surface; and
the second boss has a second contact surface for contacting and pressing the second positioning part, the second contact surface being an inclined surface or an arc surface.

12. The assembly device according to claim 10, wherein at least a portion of the first positioning part protrudes toward the pressing part relative to the first elastic part, and at least a portion of the second positioning part protrudes toward the pressing part relative to the second elastic part; and
when the pressing part moves toward the positioning assembly, the first boss is able to contact the protruding part of the first positioning part, and the second boss is able to contact the protruding part of the second positioning part.

13. The assembly device according to claim 5, wherein the first housing is connected to the first elastic part, the first housing comprises a first bearing part and a second bearing part, and the first positioning part is disposed between the first bearing part and the second bearing part;
the second housing is connected to the second elastic part, the second housing comprises a third bearing part and a fourth bearing part, and the second positioning part is disposed between the third bearing part and the fourth bearing part; and
the first bearing part and the third bearing part are arranged opposite to each other and form the receiving slot, and the second bearing part and the fourth bearing part are arranged opposite to each other and form the assembly slot.

14. The assembly device according to claim 13, wherein the first elastic part passes through the first bearing part and is provided with a first support structure, and the second elastic part passes through the second bearing part and is provided with a second support structure;
when the first elastic part is pressed, it is able to rotate around the first support structure to drive the first positioning part to move; and
when the second elastic part is pressed, it is able to rotate around the second support structure to drive the second positioning part to move.

15. The assembly device according to claim 14, wherein the first elastic part includes a first pressing plate and a first connecting rod, the first pressing plate is disposed on the side of the first bearing part away from the first positioning part, the first connecting rod passes through the first bearing part and connects the first pressing plate and the first positioning part, and the first pressing plate and/or the first connecting rod are connected to the first support structure;
the second elastic part includes a second pressing plate and a second connecting rod, the second pressing plate is disposed on the side of the third bearing part away from the second positioning part, the second connecting rod passes through the third bearing part and connects the second pressing plate and the second positioning part, and the second pressing plate and/or the second connecting rod are connected to the second support structure; and
when the first pressing plate and the second pressing plate are pressed, they are able to move closer to each other, causing the first connecting rod to rotate around the first support structure and the second connecting rod to rotate around the second support structure, thereby driving the first positioning part and the second positioning part to move away from each other.

16. The assembly device according to claim 14, wherein the end of the first elastic part away from the first positioning part is connected to the first housing, and the end of the second elastic part away from the second positioning part is connected to the second housing; and
the first support structure and the second support structure are arranged opposite to each other, and when the first elastic part and the second elastic part are pressed, the first support structure and the second support structure come into contact with each other.

17. The assembly device according to claim 14, wherein the first elastic part is connected to the first housing via the first support structure, and the second elastic part is connected to the second housing via the second support structure.

18. The assembly device according to claim 5, wherein the limiting gap between the first positioning part and the second positioning part has a first dimension L1, the mating part has a second dimension L2, and the two dimensions satisfies: L1<L2.

19. The assembly device according to any one of claims 1 to 18, wherein the receiving slot is provided with a limiting protrusion configured to limit the position of the clip assembly.

20. A hemostatic clip kit, comprising:
a clip assembly;
a delivery device; and
the assembly device according to any one of claims 1 to 19, wherein the assembly device is configured to assemble the clip assembly and the delivery device.

21. An assembly method for the assembly device according to any one of claims 1 to 19, comprising:
inserting the delivery device into the assembly slot through an opening of the housing assembly, and then into the receiving slot through the assembly slot, so that the positioning assembly of the assembly device engages with the mating part of the delivery device to position the delivery device, and the clip assembly is received in the receiving slot in a closed state;
manipulating the handle mechanism of the delivery device to insert a cable assembly of the delivery device into the clip seat of the clip assembly and connect it to the clip; and
completing the assembly by pulling the delivery device out of the assembly device and taking the clip assembly out with it.
